# EUROPEAN PATENT SPECIFICATION

(11) **EP 2 148 925 B1**
(45) Date of publication and mention of the grant of the patent: **07.03.2012**
(21) Application number: 08807124.6
(22) Date of filing: 15.05.2008
(51) Int. Cl.: C12N 15/82

(54) **METHOD FOR INCREASING THE RESISTANCE OF A PLANT TO ENDOPARASITIC NEMATODES**
VERFAHREN ZUR ERHÖHUNG DER RESISTENZ EINER PFLANZE GEGEN ENDOPARASITISCHE NEMATODEN
PROCÉDÉ POUR AUGMENTER LA RÉSISTANCE D'UNE PLANTE AUX NÉMATODES ENDOPARASITES

(30) Priority: 15.05.2007 EP 07290610
(43) Date of publication of application: 03.02.2010
(73) Proprietor: Genoplante-Valor, 75015 Paris (FR)
(72) Inventor: FAVERY, Bruno, F-06600 Antibes (FR); ABAD, Pierre, F-06800 Cagnes-sur-Mer (FR); CAILLAUD, Marie-Cécile, F-06000 Nice (FR)
(74) Representative: Rançon, Xavier Lucien Abel
(86) International application number: PCT/IB2008/002459
(87) International publication number: WO 2008/139334

(56) References cited:
- VAN DAMME DANIEL ET AL: "Molecular dissection of plant cytokinesis and phragmoplast structure: a survey of GFP-tagged proteins" PLANT JOURNAL, vol. 40, no. 3, November 2004 (2004-11), pages 386-398, XP002454156 ISSN: 0960-7412
- MÜLLER SABINE ET AL: "The plant microtubule-associated protein AtMAP65-3/PLE is essential for cytokinetic phragmoplast function." CURRENT BIOLOGY : CB 9 MAR 2004, vol. 14, no. 5, 9 March 2004 (2004-03-09), pages 412-417, XP002454157 ISSN: 0960-9822
- DATABASE WPI Section Ch, Week 200248 Thomson Scientific, London, GB; Class C12, AN 2002-449190 XP002454163 & JP 2002 125675 A (DOKURITSU GYOSEI HOJIN NOGYO SEIBUTSU SH) 8 May 2002 (2002-05-08) -& DATABASE JPO PROTEINS [Online] 17 January 2003 (2003-01-17), LIANG ET AL.: XP002454162 retrieved from EBI Database accession no. BD566254
- HUSSEY PATRICK J ET AL: "The plant cytoskeleton: Recent advances in the study of the plant microtubule-associated proteins MAP-65, MAP-190 and the Xenopus MAP215-like protein, MOR1." PLANT MOLECULAR BIOLOGY, vol. 50, no. 6, December 2002 (2002-12), pages 915-924, XP002454158 ISSN: 0167-4412
- ENGLER JANICE DE ALMEIDA ET AL: "Dynamic cytoskeleton rearrangements in giant cells and syncytia of nematode-infected roots" PLANT JOURNAL, vol. 38, no. 1, April 2004 (2004-04), pages 12-26, XP002454159 ISSN: 0960-7412 cited in the application
- FAVERY BRUNO ET AL: "Arabidopsis formin AtFH6 is a plasma membrane-associated protein upregulated in giant cells induced by parasitic nematodes" PLANT CELL, vol. 16, no. 9, September 2004 (2004-09), pages 2529-2540, XP002454160 ISSN: 1040-4651 cited in the application
- ATKINSON H J ET AL: "Engineering plants for nematode resistance" ANNUAL REVIEW OF PHYTOPATHOLOGY, ANNUAL REVIEWS INC, US, vol. 41, 1 May 2003 (2003-05-01), pages 615-639, XP002295007 ISSN: 0066-4286
- BAKHETIA M ET AL: "RNA interference and plant parasitic nematodes" TRENDS IN PLANT SCIENCE, ELSEVIER SCIENCE, OXFORD, GB, vol. 10, no. 8, August 2005 (2005-08), pages 362-367, XP004999333 ISSN: 1360-1385
- CHEN RU-GIANG ET AL: "Functional characterization of Mi, a root-knot nematode resistance gene from tomato (Lycopersicon esculentum L.)" JOURNAL OF INTEGRATIVE PLANT BIOLOGY, vol. 48, no. 12, December 2006 (2006-12), pages 1458-1465, XP002454161 ISSN: 1672-9072

## Description

The present invention relates to means for enhancing the resistance of plants to nematodes.

Plant endoparasitic nematodes are small microscopic roundworms which live in the soil and infect plant roots. They can affect a broad range of species, including vegetables, cereals, fruits, flowers, and woody plants, causing important pre- and post-harvest losses. Plant endoparasitic nematodes include for instance *Hirchmaniella* spp, *Pratylenchus* spp, *Radopholus* spp, *Ditylenchus* spp, *Anguina* spp, *Aphelenchoides* spp, *Bursaphelenchus* spp, *Rhadinaphelenchus* spp, *Heterodera* spp, *Rotylenchus* spp, *Tylenchulus* spp, *Nacobbus* and *Globodera* spp. Among the must-damaging nematodes, one can cite the root-knot nematodes *Meloidogyme* spp. These nematodes establish and maintain permanent multinucleate plant feeding cells. These giant cells are essential for this obligate biotrophic pathogen growth and reproduction. After root penetration, nematode larva intercellularly migrates to induce the dedifferentiation of five to seven vascular root cells. Selected cells tremendously enlarge and become multinucleate through synchronous nuclear divisions without complete cytokinesis (Jones and Payne, 1978). Hypertrophied mature giant cell contains more than a hundred polyploid nuclei, which have also undergone extensive endoreduplication (Wiggers *et al.,* 1990) and presents dense granular cytoplasm with numerous organelles (Jones, 1981). Surrounding giant cells, parenchyma cells hyperplasia and hypertrophy leads to typical root gall formation, the most visible infection symptom. It is not yet understood how these nematodes cause such alterations, but it is suspected that secreted parasitism proteins may have direct effects on host cells (Davis *et al.,* 2004). Nematode feeding site ontogenesis complexity is reflected by extensive gene expression modification in infected root cells (Gheysen and Fenoll, 2002; Jammes *et al.,* 2005). Genes involved in diverse processes such as cell cycle activation (de Almeida-Engler *et al.,* 1999), cell wall modification (Goellner *et al.,* 2001), hormone and defense response (Lohar *et al.*, 2004; Jammes *et al.*, 2005) have been identified. However unique example of gene function essential for giant cell formation has been demonstrated by knock out of the *rpe* gene that encodes a key enzyme in the pentose phosphate pathway (Favery *et al.,* 1998). Giant cell cytoskeleton rearrangements have also been described to be a plant key component in compatible plant-nematode interactions (de Almeida-Engler *et al.,* 2004; Favery *et al.,* 2004).

Plant microtubule associated proteins MAP65 were first purified from tobacco (*Nicotiana tabacum*) and carrot (*Daucus carota*) microtubule (MT) preparation as 65-kDa proteins (Jiang and Sonobe, 1993; Chan *et al.,* 1996). In Arabidopsis, nine genes encoding MAP65 have been identified (Hussey *et al.,* 2002). Biochemical experiments demonstrated that MAP65s bind and bundle microtubules (MTs) *in vitro* (Jiang and Sonobe, 1993; Chan *et al.,* 1996; Wicker-Planquart *et al.,* 2004). The ability to bind MTs is dependent on a conserved motif located at the protein C-terminal half (Smertenko *et al.*, 2004). Plant MAP65s share a large conserved domain with the yeast Anaphase spindle elongation factor 1 (Ase1p) and the human Protein Regulation Cytokinesis 1 (PRC1). Ase1p and PRC1 are involved in central spindle formation and cytokinesis (Pellman *et al.,* 1995; Mollinari *et al.,* 2002; Schuyler *et al.*, 2003). Different plant MAP65s may have distinct activities and functions in relation to the different MTs arrays. Transitory expression experiments in tobacco cells revealed that several members localized to the phragmoplast, but also to other MT-based structures such as the cortical MTs, preprophase band (PPB) and mitotic spindles (Van Damme *et al.,* 2004).

AtMAP65-3/PLEIADE, an AtMAP65 member, has been isolated by a genetic screen for root morphogenesis mutants (Müller *et al.,* 2002). AtMAP65-3 binds to the MTs during cell division. The *atmap65-3*/*ple* mutants display cytokinesis defects in the root meristem, presumably due to compromised phragmoplast organization (Müller *et al.,* 2004). The amino acid sequence of the AtMAP65-3 protein is available under accession number NP_199973 (or gi:15242132) in the GENBANK database. It is reproduced herein as SEQ ID NO: 2. The corresponding nucleotide sequence is also available in the GENBANK database under accession number NM_124539 (gi:30696073). It is reproduced herein as SEQ ID NO: 1. AtMAP65-3 comprises a MAP65_ASE1 domain (accession number pfam03999 in the CDD database, Marchler-Bauer and Bryant, 2004, or accession number IPR007145 in the InterPro database, Mulder *et al.,* 2007) corresponding to the amino acids 36-625 of AtMAP65-3, and also comprises an AAA (ATPase) sub-domain (accession number SM00382 in the SMART database (Schultz *et al.,* 1998 and Letunic *et al.,* 2006) corresponding to the amino acids 6-449 of AtMAP65-3.

The Inventors have investigated the molecular mechanisms underlying giant cell formation. Using a promoter trap strategy in Arabidopsis, they identified *AtMAP65-3* gene to be expressed at early stages of nematode feeding site formation. Promoter-GFP fusion analysis showed that *AtMAP65-3* was expressed in all tissues enriched in dividing cells. Dwarf *map65-3* mutants microscopical analysis revealed polynucleate and hypertrophied plant cells. In AtMAP65-3 absence, giant cells were induced but failed to fully differentiate and finally decayed. These giant cell defects lead to improper maturation of the infecting nematodes that depend on the nutrient supply from fully developed giant cell. Thus, the Inventors have shown that *AtMAP65-3* is an essential host susceptibility gene, playing a key role in plant-nematode interaction by its requirement for giant cell development.

The present invention thus provides a method for increasing the resistance of a plant to an endoparasitic nematode, wherein said method comprises the inhibition in said plant of a microtubule associated protein of the MAP65 family, hereinafter designated as MAP65-3 protein, said MAP65-3 protein having at least 50%, preferably at least 55% and by order of increasing preference, at least 58%, 60%, 65%, 70%, 75%, 80%, 85%, 90%, 95%, 98% or 99 % sequence identity, or at least 65%, preferably 70% and by order of increasing preference, at least 71%, 75%, 80%, 85%, 90%, 95%, 98% or 99% sequence similarity with the AtMAP65-3 protein (SEQ ID NO: 2), and being capable of enabling the complementation of the *Arabidopsis thaliana map65-3* mutant phenotype.

According to a preferred embodiment, said MAP65-3 protein comprises a MAP65_ASE1 domain having at least 55%, and by order of increasing preference, at least 60%, 63%, 65%, 70%, 75%, 80%, 85%, 90%, 95%, 98% or 99 % sequence identity, or at least 70%, and by order of increasing preference, at least 75%, 76%, 77%, 80%, 85%, 90%, 95%, 98% or 99% sequence similarity with the MAP65_ASE1 domain of the AtMAP65-3 protein as defined above.

According to a further preferred embodiment, said MAP65-3 protein comprises an AAA sub-domain having at least 55%, and by order of increasing preference, at least 60%, 65%, 67%, 70%, 75%, 80%, 85%, 90%, 95%, 98% or 99 % sequence identity, or at least 75%, and by order of increasing preference, at least 80%, 85%, 90%, 95%, 98% or 99% sequence similarity with the AAA sub-domain of the AtMAP65-3 protein as defined above.

Unless otherwise specified, the protein sequence identity and similarity values provided herein are calculated using the BLASTP program under default parameters, on a comparison window including the whole sequence of the proteins or of the domain to be compared. Similarity calculations are performed using the scoring matrix BLOSUM62.

By way of example of MAP65-3 proteins, one can cite the putative microtubule-associated protein of *Solanum demissum* (amino acid sequence available under accession number AAT40494 (gi: 11320514) in the GENBANK database), the microtubule-associated protein-like of *Oryza sativa* (japonica cultivar group) (amino acid sequence available under accession number NP_001043901 (gi:115439243) in the GENBANK database), or the protein of *Vitis vinifera* (amino acid sequence is available under accession number CAO49743 (gi:157355886) in the GENBANK database).

If necessary, a MAP65-3 protein can further be identified by determining its localization *in planta* (MAP65-3 locates to all microtubule arrays in dividing cells only such as the midline of the anaphase spindle and the cytokinetic phragmoplast ), *e.g.* by the methods described in Müller *et al.,* 2004, Van Damme *et al.*, 2004 and/or Caillaud *et al.,* 2008.

The complementation of the *Arabidopsis thaliana map65-3* mutant phenotype can be shown by expressing said protein in the *dyc283* mutant (see Example 1 below).

The inhibition of a MAP65-3 protein can be obtained either by abolishing, blocking or decreasing its function (*i.e.* binding to the MTs during cell division), or advantageously, by preventing or down-regulating the expression of its gene.

By way of example, inhibition of said MAP65-3 protein can be obtained by mutagenesis of the corresponding gene or of its promoter, and selection of the mutants having partially or totally lost the MAP65-3 protein activity. For instance, a mutation within the coding sequence can induce, depending on the nature of the mutation, the expression of an inactive protein, or of a protein with impaired activity; in the same way, a mutation within the promoter sequence can induce a lack of expression of said MAP65-3 protein, or decrease thereof.

Mutagenesis can be performed for instance by targeted deletion of the *MAP65-3* coding sequence or promoter, or of a portion thereof, or by targeted insertion of an exogenous sequence within said coding sequence or said promoter. It can also be performed by random chemical or physical mutagenesis, followed by screening of the mutants within the *MAP65-3* gene. Methods for high throughput mutagenesis and screening are available in the art. By way of example, one can mention TILLING (Targeting Induced Local Lesions IN Genomes, described by McCallum *et al.,* 2000).

Advantageously, the inhibition of said MAP65-3 protein is obtained by silencing of the corresponding gene. Methods for gene silencing in plants are known in themselves in the art. For instance, one can mention by antisense inhibition or co-suppression, as described by way of example in U.S. Patents 5,190,065 and 5,283,323. It is also possible to use ribozymes targeting the mRNA of said MAP65-3 protein.

Preferred methods are those wherein post transcriptional gene silencing is induced by means of RNA interference (RNAi) targeting the *MAP65-3* gene to be silenced. Various methods and DNA constructs for delivery of RNAi are available in the art (for review, cf. Watson *et al.*, 2005). Typically, DNA constructs for delivering RNAi in a plant include at least a fragment of 300 bp or more (generally 300-800 bp, although shorter sequences may sometime induce efficient silencing) of the cDNA of the target gene, under transcriptional control of a promoter active in said plant. Currently, the more widely used DNA constructs are those that encode hairpin RNA (hpRNA). In these constructs, the fragment of the target gene is inversely repeated, with generally a spacer region between the repeats.

The present invention provides chimeric DNA constructs for silencing a *MAP65-3* gene.

A chimeric DNA construct of the invention comprises:
- a promoter functional in a plant cell;
- a DNA sequence of 200 to 1000 bp, preferably of 300 to 900 bp, consisting of a fragment of a cDNA encoding a MAP65-3 protein or of its complementary, or having at least 95% identity, and by order of increasing preference, at least 96%, 97%, 98%, or 99 % identity with said fragment, said DNA sequence being placed under transcriptional control of said promoter.

According to a preferred embodiment of the invention, said chimeric DNA construct comprises:
- a first DNA sequence of 200 to 1000 bp, preferably of 300 to 900 bp, consisting of a fragment of a cDNA encoding a MAP65-3 protein, or having at least 95% identity, and by order of increasing preference, at least 96%, 97%, 98%, or 99 % identity with said fragment;
- a second DNA sequence that is the complementary of said first DNA, said first and second sequences being in opposite orientations ;
- a spacer sequence separating said first and second sequence, such that these first and second DNA sequences are capable, when transcribed, of forming a single double-stranded RNA molecule.

The spacer can be a random fragment of DNA. However, preferably, one will use an intron which is spliceable by the target plant cell. Its size is generally 400 to 2000 nucleotides in length.

A large choice of promoters suitable for expression of heterologous genes in plants is available in the art.

They can be obtained for instance from plants, plant viruses, or bacteria such as *Agrobacterium.* They include constitutive promoters, *i.e.* promoters which are active in most tissues and cells and under most environmental conditions, tissue or cell specific promoters which are active only or mainly in certain tissues (*e.g.* root cells) or certain cell types (*e.g.* giant cells), and inducible promoters that are activated by physical or chemical stimuli, such as those resulting from nematode infection.

One can use constitutive promoters that are commonly used in plant cells are the cauliflower mosaic virus (CaMV) 35S promoter, the Nos promoter, the rubisco promoter.

However, the most suitable promoters for silencing the *MAP65-3* gene in a plant in order to increase its resistance to an endoparasitic nematode are promoters that are tissue-specific, having a preferential or exclusive expression in the tissues infected by the nematodes, in particular the giant cells, and/or promoters that are inducible by nematode infection, *i.e.* promoters that drive an increased level of expression in cells infected by nematodes, when compared to non-infected cells. Said promoters may be naturally occurring promoter, may comprise a nematode responsive element isolated from a naturally occurring promoter, or may be a synthetic promoter.

Non-limitative examples of preferred promoters for carrying out the invention include:
- the elongation specific endo-1,4-.beta.-glucanase (cell) promoter of *Arabidopsis thaliana*, which is described in U.S. Patent No. 7,119,254 as being up-regulated in root-knot nematode feeding cells;
- the promoter of the POX gene of *A. thaliana* (Vercauteren *et al.,* 2001);
- the promoter of the Trypsin inhibitor gene of *A. thaliana* (Vercauteren *et al.,* 2001);
- the promoter of the endomembrane protein gene of *A. thaliana* (Vercauteren *et al.*, 2001);
- the promoter of the DAP decarboxylase gene of *A. thaliana* (Vercauteren *et al.*, 2001);
- the promoter of the WRKY23 (Att0001) gene of *A. thaliana* (Barthels *et al.,* 1997); or preferably
- the promoter of the *AtFH6* gene (Favery *et al.,* 2004), also disclosed in WO 2005/063989 (SEQ ID NO: 1 of WO 2005/063989).

The DNA constructs of the invention generally also include a transcriptional terminator (for instance the 35S transcriptional terminator, or the nopaline synthase (Nos) transcriptional terminator).

These DNA constructs can be obtained and introduced in a host cell or organism by the well-known techniques of recombinant DNA and genetic engineering. The choice of the recombinant vector depends on the intended host and on the intended method of transformation of said host. A variety of methods for genetic transformation of plant cells or plants are available in the art for many plant species, dicotyledons or monocotyledons. By way of non-limitative examples, one can mention virus mediated transformation, transformation by microinjection, by electroporation, microprojectile mediated transformation, *Agrobacterium* mediated transformation, and the like.

The invention also comprises plant cells or plants genetically modified by a construct of the invention. The polynucleotide may be transiently expressed; it can also be incorporated in a stable extrachromosomal replicon, or integrated in the chromosome.

In particular the invention relates to a transgenic plant containing a transgene comprising a chimeric DNA construct of the invention. The expression of said chimeric DNA constructs, resulting in a down regulation of the MAP65-3 protein, provides to said transgenic plant an increased resistance to endoparasitic nematodes when compared with a plant devoid of said transgene.

The present invention applies to monocot- or dicotyledon plants of agronomical interest, such as wheat, maize, rice, oats, barley, rye, sorghum, triticale, sugar cane, tomato, potato, sugarbeet, rape, flax, tobacco, sunflower, cotton, peas, beans, soybean or alfalfa.

The invention also encompasses isolated organs or tissues of said plants (such as seeds, leafs, flowers, roots).

Foregoing and other objects and advantages of the invention will become more apparent from the following detailed description and accompanying drawings. It is to be understood however that this foregoing detailed description is exemplary only and is not restrictive of the invention.

### DESCRIPTION OF THE DRAWINGS:

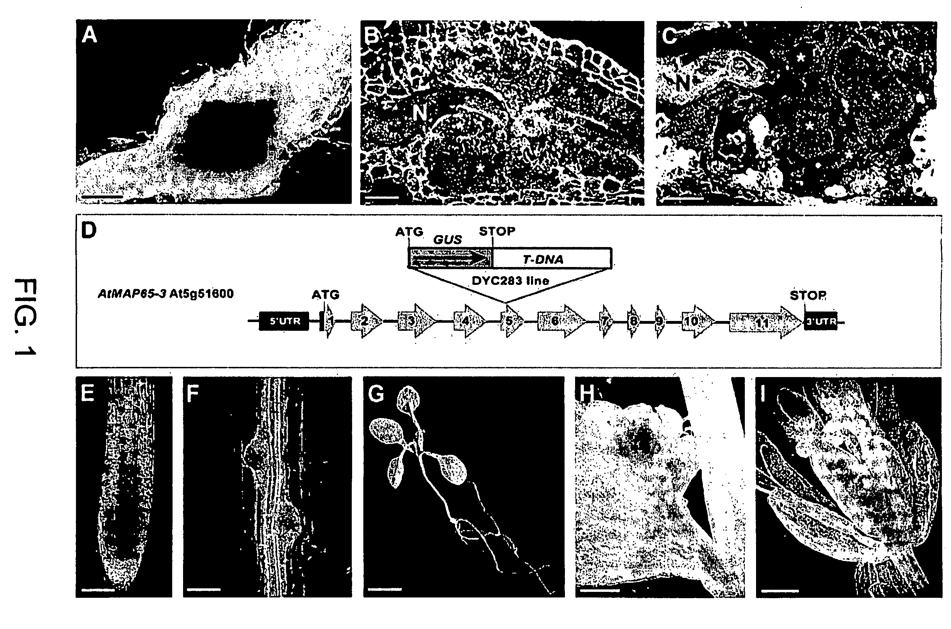

**Figure 1****.** DYC283 T-DNA-Tagged Arabidopsis Line Displaying GUS Activity in Galls Induced by *M. incognita* and during Plant Development. **(A-C)** GUS expression in galls induced by *M. incognita.* Localized GUS activity in a root gall 7 days post infection (dpi) **(A)**. Sectioned gall shown in **(A)** seen by dark-field microscopy **(B)**. GUS activity (seen as a pink precipitate) is observed in the giant cells and in the surrounding cells. Sectioned galls 21 dpi observed in dark-field microscopy. Asterisks, giant cells; N, nematode. **(C)**. GUS activity is observed in surrounding cells and not in mature giant cells. Asterisks, giant cells; N, nematode. **(D)** Organization of the *AtMAP65-3* gene and molecular analysis of the T-DNA insertion. Boxes indicate the exons. GUS corresponds to the coding region of the β-glucuronidase gene present on the T-DNA. The initiation and stop codons are indicated. **(E-I)** GUS expression during plant development. Reporter gene activity was observed in root meristem **(E)**, lateral root meristem **(F)** developing leaves of a 14-day-old seedling **(G)**, buds **(H)** and ovules **(I)**. Bars in **(A)** = 100 µm; **(B)** and **(C)** = 25 µm; in **(E)** and **(F)** = 50 µm; in **(H)** and **(I) =** 150 µm, in **(G)** = 1 cm.

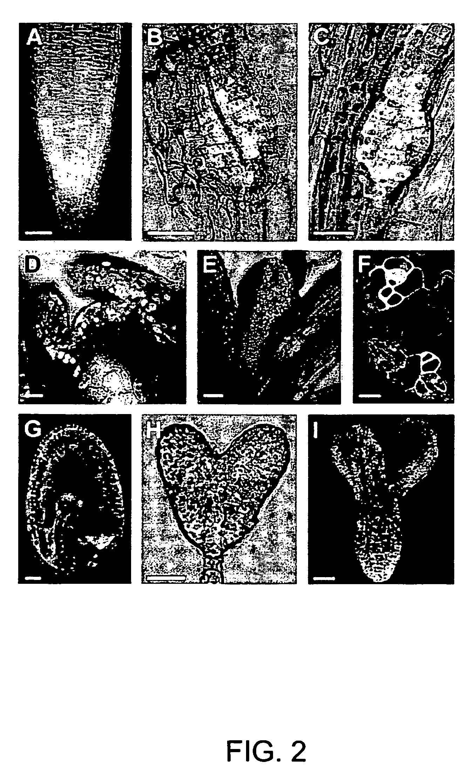

**Figure 2****.** Gene Expression Patterns Observed in AtMAP65-3 Promoter:GFP:GUS Fusion Line. **(A-C)** GFP expression in the root, in root meristem **(A),** lateral root emergency **(B)** and lateral root meristem **(C). (D-F)** GFP expression in the shoot, in the shoot apical meristem **(D),** young leaves **(E).** In mature leaves GFP signal is located in meristemoids, stomata in formation and in mature stomata **(F). (G-1)** GFP expression during embryogenesis, in endosperm and embryo **(G),** in heart embryo stage **(H)** and in late torpedo stage **(I).** Bars in **(A)** to **(D)** and **(G)** to **(I)** = 50 µm, in **(E)** = 200 µm, in **(F)** = 10 µm.

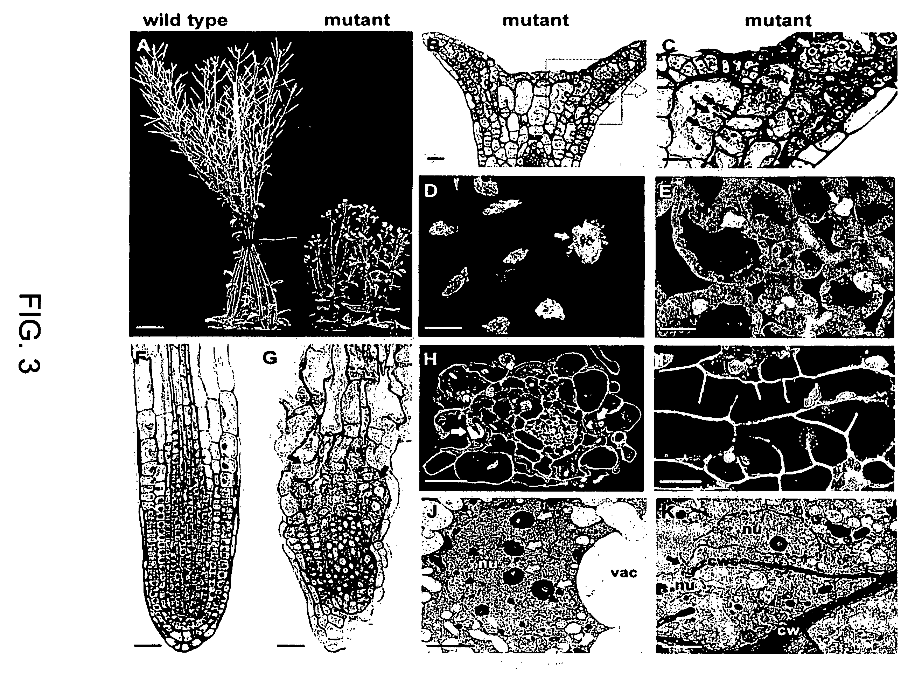

**Figure 3****.** *AtMAP65-3* Interruption Leads to a Shoot and Root Mutant Phenotype. **(A-E)** *dyc283* mutant shoot phenotype in plants homozygous for the T-DNA mutation (*dyc283*/*dyc283*). The *dyc283* mutant showed a strong reduction of the shoot in comparison with wild type plant **(A).** Hypocotyl and leaf primordial **(B-C)** exhibited polynucleate cells and aberrant cell wall stubs (arrows). In leaf primordia, nuclei were often irregular lobed and amoeboid in form **(D).** Mature leaf stained with DAPI, major part of mesophyll cells was binucleate and some cells presented four nuclei **(E)** (arrows). **(F-K)** *dyc283* mutant root phenotype. Longitudinal section through wild type root **(F)**. Longitudinal section through *dyc283* mutant revealed multinucleate hypertrophied cortical and epidermal cells (arrows) **(G).** Transversal section through mutant root stained with DAPI showed polynucleate (arrows) and hypertrophied cells **(H).** Vascular cylinder cells often show cell misalignments (arrows) **(H).** *dyc283* mutant expressing H2B:YFP and stained with membrane dye FM4-64 exhibited multinucleate root cells and cell wall stubs **(I).** *dyc283* mutant root electron myograph showed abnormal enlarged nuclei, that contained nucleolus number increase (arrows) **(J),** non-divided nucleus (arrow) curving around incomplete wall branched end **(K).** nu, nucleus; vac, vacuole; cws, cell wall stub; cw, cell wall. Bars in **(A)** = 2 cm; in **(B), (F)** and **(G)** = 50 µm; in **(C)** to **(E), (H)** and **(I)** = 20 µm; in **(J)** and **(K)** = 1 µm.

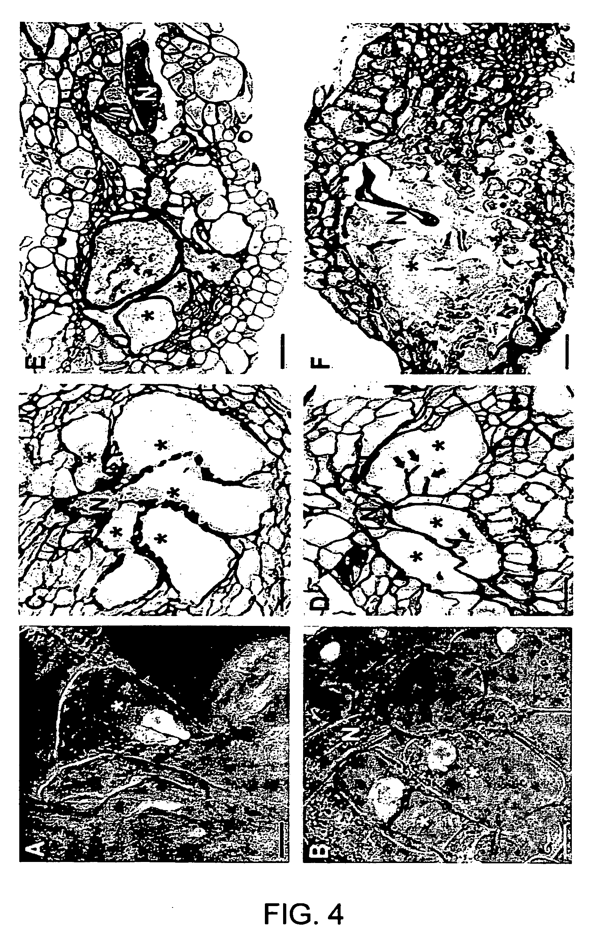

**Figure 4****.** Giant Cells and Root-Knot Nematode Mutant Phenotype **(A-B)** Visualization of nuclei in young giant cells using plant expressing H2B:YFP. In control wild-type plant, giant cell was binucleate **(A).** In *dyc283* mutant plant, giant cells were mononucleate with swollen nuclei **(B). (C-D)** Cross section through gall of wild-type plant **(C)** and *dyc283* plant **(D),** 7 dpi with *M. incognita.* Arrows showed aberrant cell wall stubs in *dyc283* mutant giant cell **(D). (E-F)** Cross section through gall of wild type plant **(E)** and *dyc283* mutant plant **(F),** 21 dpi with *M. incognita.* In control wild-type plant, giant cells were mature and nematode was at the fourth juvenile stage **(E).** In the *dyc283* mutant plant, giant cells decayed and nematode stayed at the third stage **(F).** Asterisks, giant cells; N, nematode. Bars in **(A)** to **(D)** = 20 µm; in **(E)** and **(F)** = 40 µm.

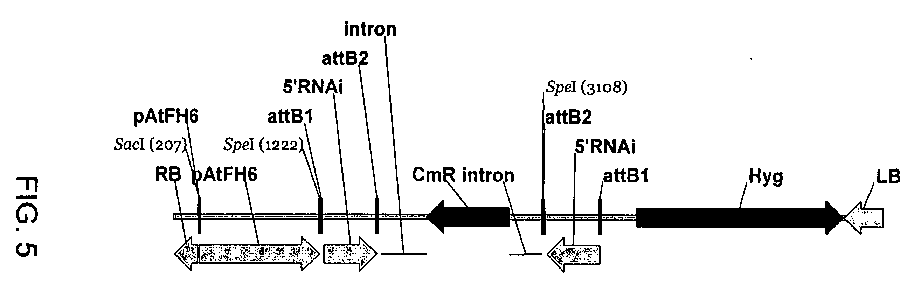

**Figure 5****.** Pro_{AtFH6}-RNAi AtMAP65-3 T-DNA construct used for the generation of transgenic plants with the *MAP65-3* gene silenced in giant cells.

### EXAMPLES

### EXPERIMENTAL PROCEDURES

### Plant materials, growth conditions, and nematode infection

The T-DNA mutagenized *Arabidopsis thaliana* line collection (ecotype Wassilewskija, WS) was generated at Institut National de la Recherche Agronomique Versailles for promoter trap and gene tagging (Bechtold *et al.,* 1993). The lines were screened individually for GUS expression after *Meloidogyne incognita* infection as previously described (Favery *et al.,* 1998). For *in vitro* analyses, seeds were surface sterilized and grown on MS medium containing 1% sucrose, 0.7% plant cell culture tested agar (Sigma, St. Louis, MO, USA), and 50 µg/mL of kanamycin. Plates were inclined at an angle of 60° to allow the roots to grow along the surface. Kanamycin resistance was scored in 2-week-old seedlings. For nematode infection *in vitro,* 100 surface-sterilized freshly hatched *M. incognita* J2 were added on each 2-week-old seedling. The plates were kept at 20°C with a 16-h photoperiod.

### Histochemical localization of GUS activity and microscopic analyses

GUS activity was assayed histochemically with 5-bromo-4-chloro-3-indolyl-β-D-glucuronic acid as described by Favery *et al.* (1998). Galls, root apex, and shoot apical meristems were dissected from GUS-stained plants, fixed in 1% glutaraldehyde and 4% formaldehyde in 50 mM sodium phosphate buffer, pH 7.2, dehydrated, and embedded in Technovit 7100 (Heraeus Kulzer, Wehrheim, Germany) as described by the manufacturer. Sections (4 µm) were stained with 0.05% ruthenium red or toluidine blue and mounted in DPX (BDH Laboratory Supplies, VWR International, Fontenay-sous-Bois, France). Sections were observed with a Zeiss Axioplan 2 microscope (Jena, Germany).

### T-DNA insertion site analysis and isolation of homozygous dyc283 and ebj96 plants

The insertion site was characterized by sequencing the genomic regions flanking the inserted T-DNA as described by Samson *et al.* (2002). To isolate homozygous dyc283/dyc283 and ebj96/ebj96 plants, the segregation of the kanamycin marker carried by the T-DNA on progenies resulting from each of 20 plants was analyzed. Progenies of five plants segregated 100% kanamycin-resistant plants, indicating that they were homozygous for the T-DNA tagged allele. To confirm these results, PCR experiments were done with the *AtAMP65-3* primers which span the T-DNA insertion site, and a third primer GUS (5'-tcc aga ctg aat gcc cac ag-3', SEQ ID NO: 3) specific for the sequence of the T-DNA. The primers DYCR (5'-gca gtt cag aag ctg atg gag g-3', SEQ ID NO: 4) and DYC5RC (5'-cct gcc tga gta tgt tat act cc-3', SEQ ID NO: 5) were used for DYC283, and DYC6 (5'-gga gta taa cat act cag gca gg-3', SEQ ID NO: 6) and DYC9RC (5'-gat gat caa acc aaa cga cat tca g-3', SEQ ID NO: 7) for EBJ96. When genomic DNA from homozygous plants was used as a template, a single PCR product was obtained from amplifications with all three primers.

### Transgenic plants and crosses

For AtMAP65-3 promoter GFP:GUS fusion, a fragment of 1240 bp upstream of the start codon was amplified by PCR using the primers Gw5pdyc (5'-aaa aag cag gct tca cac tct tcc cta cac aaa acc gc-3', SEQ ID NO : 8) and Gw3pdyc (5'-aga aag ctg ggt gtt cga aat gct taa gcc tgt aac agg g-3', SEQ ID NO : 9). The PCR fragment was inserted into the pDON207 donor vector and then in the plant expression vectors pKGWFS7 (Karimi *et al.,* 2002) using Gateway Technology (Invitrogen). For the subcellular localisation of MAP65-3, the Pro_{35S} HindIII / SpeI fragment of the pK7WGF2, pK7FWG2, pH7WGY2, pH7YWG2 vectors (Karimi *et al.,* 2002) was replaced by Pro_{MAP65-3}. The coding sequence of MAP65-3 was amplified by PCR, using wild-type plant cDNAs as the template and the primers Gw5dycB (5'-aaa aag cag gct tca cca tgg caa gtg ttc aaa aag atc cg-3', SEQ ID NO : 10) and Gw3dycK (5'-aga aag ctg ggt gtc aaa cca aac gac att cag act g-3', SEQ ID NO : 11) or Gw3dycL (5'-aga aag ctg ggt gaa cca aac gac att cag act g-3', SEQ ID NO : 12) for GFP or YFP:AtMAP65-3 or AtMAP65-3:GFP or YFP fusion respectively. These sequences were inserted into the pDON207 donor vector and then in the Pro_{MAP65-3} plant expression vectors using Gateway Technology (Invitrogen). These constructs were sequenced by Genome Express (Grenoble, France) and transformed into *Agrobacterium tumefaciens* strain GV3101. Wild type WS, homozygous dyc283 or ebj96 Arabidopsis plants were transformed using the dipping method (Clough and Bent, 1998) and selected on MS medium 0.7% agar plates containing 50 µg/ml kanamycin or hygromycin. Transformed plants were transferred to soil and seeds collected. Transgenic plants expressing Pro_{MAP65-3}:AtMAP65-3:GFP or the N-terminal domain of the MT-binding domain of MAP4 fused to the GFP (Pro_{35S}:MBD:GFP) were crossed with Pro_{35S}:H2B:YFP Arabidopsis plants. Plants expressing the two constructions were obtained and homozygous progeny was used for microscopy analysis.

### Confocal microscopy

Optical sections were obtained on fresh roots by using an inverted confocal microscope (model LSM510; Zeiss, Jena, Germany). To visualize the MT cytoskeleton and nuclei in nematode feeding site, galls of 7 and 14 dpi were excised and embedded in agar 7%. Vibroslices of 100 µm diameter- for galls 7 dpi- or 300 µm diameter -for galls 14 dpi- were obtained using a vibratome HM560V (Microm). Fresh roots and vibroslices were observed with a × 63 water immersion apochromat objective (Zeiss, Jena, Germany). YFP and GFP fluorescence were monitored in Lambda mode with a 499-550-nm beam path (488-nm excitation line). Fluorescent dye FM4-64 (Molecular probes) was used at 1 µM final concentration. GFP or YFP and FM4-64 fluorescence were monitored in Lambda mode with a 499-620-nm beam path (488-nm excitation line).

### EXAMPLE 1: THE DYC283-TAGGED LINE DISPLAYED GUS ACTIVITY IN THE NEMATODE FEEDING SITE

To isolate genes involved in giant cell formation induced by *Meloidogyne incognita*, a promoter trap strategy was developed. 20,000 T-DNA-tagged Arabidopsis lines were screened by GUS assay after root-knot nematode infection, and lines showing GUS induction in root galls (Favery *et al.,* 2004) were identified. One of these lines, DYC283, displayed early GUS activity in galls (Figure 1A), that was detected less than 48 h after giant cell initiation. Sections through galls 7 days post infection (dpi) clearly showed GUS staining in developing giant cells and in dividing surrounding cells (Figure 1 B). Later during the interaction, 21 dpi, GUS expression was detected only in surrounding cells and disappeared in mature giant cells (Figure 1C). DYC283 line molecular and genetic analysis showed that this line carried a single T-DNA insert (data not shown). The insertion site was characterized by sequencing the genomic regions flanking the inserted T-DNA (FST project, Samson *et al.*, 2002). Sequence analysis, using the Arabidopsis Resource (TAIR), showed that the T-DNA had integrated into the predicted At5g51600 gene, named *AtMAP65-3.* This gene has 11 exons and encodes a 707 amino acid long protein AtMAP65-3, which is a member of the MT-associated protein MAP65 family (Müller *et al.*, 2004). In the DYC283 line, the T-DNA was inserted into the fifth exon, placing the GUS gene ATG in frame with *AtMAP65-3* gene and resulting in a functional gene fusion (Figure 1D).

### EXAMPLE 2: ATMAP65-3 IS EXPRESSED IN ALL TISSUES ENRICHED IN DIVIDING CELLS

During plant development, the GUS gene was expressed in the root meristem and in the root elongation zone, in which cells divide and expand (Figure 1E). GUS activity was also detected in the lateral root primordia (Figure 1F). In aerial parts of the plant, GUS expression was observed in young leaves, buds and flowers (Figure 1G-I). The AtMAP65-3 expression pattern deduced from experiments with the GUS reporter gene was confirmed using RT-Q-PCR analysis (data not shown) and *AtMAP65-3* promoter *(Pro_{MAP65-3})* GFP fusion. Arabidopsis plants transformed with a *Pro_{MAP65-3}: GFP:GUS* construct presented an expression pattern in accordance with the GUS expression observed in DYC283 line (Figure 2A-E). GFP expression pattern analysis showed that *AtMAP65-3* was expressed in all tissues enriched in dividing cells, such as shoot apical meristem (SAM) (Figure 2D), foliar primordia and young leaves (Figure 2E). In older leaves, expression was restricted to meristemoid and mature stomata (Figure 2F). During embryogenesis, GFP signal was observed at early stage post fertilization, in the embryo and in the syncytial endosperm (Figure 2G). In torpedo stage, GFP signal was uniform (Figure 2H) whereas in late torpedo stage, GFP signal was observed through more-intense staining in the zone corresponding to root meristem (Figure 2I).

### EXAMPLE 3: ATMAP65-3 INTERRUPTION LEADS TO POLYNUCLEATE AND HYPERTROPHIED PLANT CELLS

Plants homozygous for the T-DNA mutation (*dyc283* mutant) showed a strong reduction of the shoot part of the plant (Figure 3A). Despite this dwarf phenotype, no organ fusions or abnormal organ numbers have been observed. *dyc283* mutant was fertile, but the number of seeds per silique was strongly reduced. Microscopical analysis revealed that the embryo sac decayed at early steps after fertilization (data not shown).

*dyc283* mutant cellular organization was characterized by performing sections through different tissues. *dyc283* mutant SAM presented irregular cell outer layers. Hypocotyl and leaf primordia exhibit polynucleate cells and aberrant cell wall stubs (Figure 3B-C), absent in wild type. In young tissues, like in leaf primordial, nuclei were often irregular lobed and amoeboid in form (Figure 3D). In mature leaves, major part of mesophyll cells was binucleate and some cells presented, at least, four nuclei (Figure 3E). Cell wall stubs and polynucleate cells were also observed during embryogenesis in *dyc283* mutant line. *dyc283* mutant has also a short and swollen root system with few lateral roots (Figure 3F and 3G). Section through elongation zone revealed polynucleate hypertrophied cortical and epidermal cells (Figure 3H). Vascular cylinder cells, despite being less affected by the mutation, often showed cell misalignments. Root cells were often multinucleate and exhibited cell wall stubs (Figure 3H and 31). Electron microscopy performed in *dyc283* mutant root confirmed presence of abnormal enlarged nuclei that contained nucleolus number increase (Figure 3J). In addition, some *dyc283* mutant cells exhibited non-divided nucleus curving around incomplete wall branched end (Figure 3K). Organ cross sections performed in DYC283 heterozygous plants revealed no aberrant cellular phenotype confirming the recessive nature of the mutation (data not shown). Thus *dyc283* mutant showed a cytokinesis defect characterized by cell wall stubs, polynucleate and hypertrophied cells in all plant organs.

### EXAMPLE 4: IN ABSENCE OF ATMAP65-3, GIANT CELLS WERE INDUCED BUT FAILED TO FULLY DIFFERENTIATE

*dyc283* mutant response to the nematode *M. incognita* was examined. The infective second stage juvenile (J2) was able to invade the *dyc283* mutant root tissue by penetrating the elongation zone, migrating along the vascular cylinder and induce a gall, as well as in wild type plant. Observation of gall sections 7 dpi revealed that the nematode could initiate its feeding site composed of 5 to 7 giant cells (Figure 4A). At this stage, giant cells in the *dyc283* mutant were slightly smaller than wild type (Figure 4B), and were characterized by unusual cell wall stubs (Figure 4A). At the early step of giant cell initiation, unique enlarged nucleus is observed in the *dyc283* mutant giant cell (Figure 4C) when wild type giant cells were binucleate (Figure 4D). Once the nematode feeding site was initiated, J2 stage nematodes became sedentary and developed into third stage juvenile. Later during the interaction, when nematodes develop into the fourth developmental stage in wild type, *dyc283* mutant gall sections revealed that nematode development was arrested at the third stage juvenile. No fourth juvenile stage has ever been observed in *dyc283* mutant. Degenerated giant cells and dead nematodes were observed 21 dpi (Figure 4E). Mature giant cells have never been found as observed in wild type (Figure 4F). Consequently, nor female, nor egg masses could be observed on the *dyc283* mutant root surface. This incomplete root-knot nematode development indicated a defect in *dyc283* mutant giant cell formation.

### EXAMPLE 5: ATMAP65-3:GFP FUSIONS COMPLEMENT THE MUTANT PHENOTYPE

Searching Arabidopsis mutant collections for loss-of-function insertion mutants, an additional *map65-3* allele was identified from INRA Versailles (Samson *et al.*, 2002). The EBJ96 line carried a single T-DNA insertion in the *AtMAP65-3* eleventh exon. As expected, plants homozygous for the *ebj96* mutation exhibited similar mutant phenotype to *dyc283* mutant. To complement the mutant phenotype, two *AtMAP65-3:GFP* fusions, *GFP:AtMAP65-3* and *AtMAP65-3:GFP*, were constructed under the *Pro_{MAP65-3}* control. When introduced into *dyc283* or *ebj96* mutants both constructs restored wild type phenotype. These results demonstrated that the *map65-3* recessive mutation was responsible to the observed phenotype.

### EXAMPLE 6: GENERATION OF TRANSGENIC PLANTS WITH THE MAP65-3 GENE SILENCED

For AtMAP65-3 specific silencing in giant cells, the Pro_{35S} SacI / SpeI fragment of the pH7GWIWG2(II) vectors (VIB Gent, Karimi *et al.,* 2002) was replaced by the promoter of AtFH6. The 449 bp 5'UTR-exonI sequence of MAP65-3 (SEQ ID NO : 13) was amplified by PCR, using wild-type plant genomic DNA as the template and the primers MAP_RNAiGW5 (5'- aaa aag cag gct tcc cca aaa cct ttt act tct tcg -3', SEQ ID NO : 14) and MAP_RNAiGW3 (5'- aga aag ctg ggt ggg agt tcg aat aaa aga gat cca -3', SEQ ID NO : 15) - AtMAP65-3 specific sequence underlined. This sequence was inserted into the pDON207 donor vector and then in the Pro_{AtFH6}-pH7GWIWG2(II) vector using Gateway Technology (Invitrogen). The Pro_{AtFH6}-RNAi AtMAP65-3 construct (Figure 5) was sequenced and transformed into *Agrobacterium tumefaciens* strain GV3101. Pro_{MAP66-3}:GFP-GUS plants were transformed using the dipping method (Clough and Bent, 1998) and selected on MS medium 0.7% agar plates containing 50 µg/ml hygromycin. Transformed plants are transferred to soil and seeds collected. The efficiency of AtMAP65-3 silencing in roots is scored using GUS staining and GFP microscopy observation of transformed PrO_{MAP65-3}:GFP-GUS plants.

T2 plants from 19 T1 transformants obtained with the Pro_{AtFH6}-RNAi AtMAP65-3 construct showed a significant reduction of the GUS expression limited to the galls induced by the nematodes. As expected, the AtFH6 promoter being not activated in the root meristems, no reduction of the GUS activity was observed in the root meristems and no developmental phenotype was detected. The first test of infection by the root knot nematodes performed on the T2 plants coming from 10 T1 plants bearing the Pro_{MAP65-3}:GFP-GUS and Pro_{AtFH6}-RNAi AtMAP65-3 constructs allowed identifying a line with a reduction of 60 % of the number of nematodes able to carry out their development cycle (2 months after infection).

### REFERENCES

Barthels et al., Plant Cell 9, 2119-2134 (1997).
Bechtold et al., C.R. Acad. Sci. Paris 316, 1194-1199 (1993).
Caillaud et al., Plant Cell 20, 423-437 (2008).
Chan et al., Plant J. 10, 251-259 (1996).
Clough and Bent, Plant J. 16, 735-43 (1998).
Davis et al., Trends Parasitol. 20, 134-141 (2004).
de Almeida-Engler et al., Cell 11, 793-807 (1999).
de Almeida-Engler et al., Plant J. 38, 12-26 (2004).
Favery et al., Embo J. 17, 6799-6811 (1998).
Favery et al., Plant Cell 16, 2529-2540 (2004).
Gheysen and Fenoll, Annu. Rev. Phytopathol. 40, 191-219 (2002).
Goellner et al., Plant Cell 13, 2241-2255 (2001).
Hussy et al., Plant Mol. Biol. 50, 915-924 (2002).
Jammes et al., Plant J. 44, 447-458 (2005).
Jiang and Sonobe, J. Cell Sci. 105, 891-901 (1993).
Jones, New York: Academic Press, pp. 225-279 (1981).
Jones and Payne, J. Nematol. 10, 70-84 (1978).
Karimi et al., Trends Plant Sci. 7, 193-195 (2002).
Letunic et al., Nucleic Acids Res 34, D257-D260 (2006).
Lohar et al., Plant J. 38, 203-214 (2004).
Marchler-Bauer and Bryant, Nucleic Acids Res. 32, 327-331 (2004).
McCallum et al., Plant Physiol., 123, 439-442 (2000).
Mollinari et al., J. Cell Biol. 157, 1175-1186 (2002).
Mulder et al., Nucleic Acids Res. 35 (Database Issue),D224-228 (2007).
Müller et al., Plant Physiol. 130, 312-324 (2002).
Müller et al, Curr. Biol. 14, 412-417 (2004).
Pellman et al, J. Cell Biol. 130, 1373-1385 (1995).
Samson et al., Nucleic Acids Res. 30, 94-97 (2002).
Schultz et al., Proc. Natl. Acad. Sci. USA 95, 5857-5864 (1998).
Schuyler et al., J. Cell Biol. 160, 517-528 (2003).
Smertenko et al., Plant Cell 16, 2035-2047 (2004).
Van Damme et al., Plant J. 40, 386-398 (2004).
Vercauteren et al., Mol Plant Microbe Interact 14, 288-299 (2001).
Watson et al., FEBS Letters, 579, 5982-5987 (2005).
Wicker-Planquart et al., Plant J. 39, 126-134 (2004).
Wiggers et al., Phytopathology 80, 1391-1395 (1990).

## Claims

1. A method for increasing the resistance of a plant to an endoparasitic nematode, wherein said method comprises the inhibition in said plant of a microtubule associated protein of the MAP65 family, said protein hereinafter designated as MAP65-3 protein, having at least 50% sequence identity or at least 71% sequence similarity with the AtMAP65-3 protein of SEQ ID NO: 2, and being capable of enabling the complementation of the *Arabidopsis thaliana map65-3* mutant phenotype.

2. A method according to claim 1, wherein the inhibition of said MAP65-3 protein is obtained by expressing in said plant RNAi targeting the gene encoding said protein.

3. A chimeric DNA construct for silencing a *MAP65-3* gene comprising:
- a promoter functional in a plant cell;
- a DNA sequence of 200 to 1000 bp, selected among:
a) a fragment of a cDNA encoding a MAP65-3 protein as defined in claim 1, or of the complementary of said cDNA, or
b) a fragment of DNA having at least 95% identity with said fragment a);
said DNA sequence being placed under transcriptional control of said promoter.

4. A chimeric DNA construct of claim 3, comprising:
- a first DNA sequence of 200 to 1000 bp, consisting of a fragment of a cDNA encoding a MAP65-3 protein as defined in claim 1, or having at least 95% identity with said fragment;
- a second DNA sequence that is the complementary of said first DNA, said first and second sequences being in opposite orientations;
- a spacer sequence separating said first and second sequences, such that these first and second DNA sequences are capable, when transcribed, of forming a single double-stranded RNA molecule.

5. A chimeric DNA construct of any of claims 3 or 4, wherein the promoter is a nematode inducible promoter.

6. A recombinant vector comprising a chimeric DNA construct of any of claims 3 to 5.

7. A transgenic plant containing a transgene comprising a chimeric DNA of any of claims 3 to 5.

## Patentansprüche

1. Verfahren zur Erhöhung der Resistenz einer Pflanze gegen einen endoparasitischen Nematoden, wobei das Verfahren die Inhibition eines Mikrotubulus-assoziierten Proteins der MAP65-Familie in der Pflanze umfasst, und das Protein, das nachstehend als MAP65-3-Protein bezeichnet wird mindestens 50% Sequenzidentität oder mindestens 71% Sequenzähnlichkeit mit dem AtMAP65-3-Protein der SEQ ID NO: 2 hat und dazu befähigt ist, die Komplementation des *Arabidopsis thaliana map 65-*3-Mutantenphänotyps zu ermöglichen.

2. Verfahren gemäß Anspruch 1, wobei die Inhibition des MAP65-3-Proteins durch Exprimieren von RNAi in der Pflanze erzielt wird, abzielend auf das Gen, das das Protein codiert.

3. Chimäres DNA-Konstrukt zur Stummschaltung eines *map65-3-*Gens, umfassend
- einen in einer Pflanzenzelle funtkionalen Promotor;
- eine DNA-Sequenz von 200 bis 1000 bp, ausgewählt aus:
a) einem Fragment einer cDNA, die ein MAP65-3-Protein, wie definiert in Anspruch 1, codiert, oder einer komplementären cDNA davon, oder
b) einem DNA-Fragment, das mindestens 95% Identität mit dem Fragment a) hat;
und wobei die DNA-Sequenz unter transkriptionelle Kontrolle des Promotors gestellt ist.

4. Chimäres DNA-Kontrukt gemäß Anspruch 3, umfassend
- eine erste DNA-Sequenz von 200 bis 1000 bp, bestehend aus einem Fragment einer cDNA, die ein MAP65-3-Protein, wie definiert in Anspruch 1, codiert, oder mindestens 95% Identität zu dem Fragment hat;
- eine zweite DNA-Sequenz, die komplementär zu der ersten DNA ist, und wobei die erste und zweite Sequenz in entgegengesetzten Orientierungen vorliegen;
- eine Abstandshalter-Sequenz, die die erste und zweite Sequenz voneinander trennt, sodass diese erste und zweite DNA-Sequenz dazu fähig sind, ein einziges doppelsträngiges RNA-Molekül zu bilden, wenn sie transkribiert werden.

5. Chimäres DNA-Konstrukt gemäß einem der Ansprüche 3 oder 4, wobei der Promotor ein Nematoden-induzierbarer Promotor ist.

6. Rekombinanter Vektor, der ein chimäres DNA-Konstrukt gemäß einem der Ansprüche 3 bis 5 umfasst.

7. Transgene Pflanze, die ein Transgen enthält, das eine chimäre DNA gemäß einem der Ansprüche 3 bis 5 umfasst.

## Revendications

1. Procédé permettant d'augmenter la résistance d'une plante à un nématode endoparasite, dans lequel ledit procédé comprend l'inhibition dans ladite plante d'une protéine associée à une microtubule de la famille MAP65, ladite protéine désignée ci-après comme étant la protéine MAP65-3, ayant au moins 50% d'identité de séquence ou au moins 71% de similarité de séquence avec la protéine AtMAP65-3 de la SEQ. ID N°2, et capable de permettre la complémentation du phénotype mutant *Arabidopsis thaliana map 65-3.*

2. Procédé selon la revendication 1, dans lequel l'inhibition de ladite protéine MAP65-3 est obtenue en exprimant dans ladite plante l'ARNi ciblant le gène codant ladite protéine.

3. Produit de synthèse d'ADN chimérique pour inhiber un gène MAP65-3 comprenant :
- un promoteur fonctionnel dans une cellule végétale ;
- une séquence d'ADN de 200 à 1000 bp, choisie parmi :
a) un fragment d'ADNc codant une protéine MAP65-3 telle que définie dans la revendication 1, ou complémentaire dudit ADNc, ou
b) un fragment d'ADN ayant au moins 95% d'identité avec ledit fragment a) ;
ladite séquence d'ADN étant placée sous un contrôle transcriptionnel dudit promoteur.

4. Produit de synthèse d'ADN chimérique selon la revendication 3, comprenant :
- une première séquence d'ADN de 200 à 1000 bp, constituée d'un fragment d'un ADNc codant une protéine MAP65-3, telle que définie dans la revendication 1, ou ayant au moins 95% d'identité avec ledit fragment ;
- une seconde séquence d'ADN qui est complémentaire dudit premier ADN, lesdites première et seconde séquences étant dans une orientation opposée ;
- une séquence d'espacement séparant lesdites première et seconde séquences, de sorte que ces première et seconde séquences d'ADN soient capables, lorsqu'elles sont transcrites, de former une molécule d'ARN double brin unique.

5. Produit de synthèse d'ADN chimérique selon l'une quelconque des revendications 3 ou 4, dans lequel le promoteur est un promoteur inductible de nématode.

6. Vecteur recombinant comprenant un produit de synthèse d'ADN chimérique selon l'une quelconque des revendications 3 à 5.

7. Plante transgénique contenant un transgène comprenant un ADN chimérique selon l'une quelconque des revendications 3 à 5.


### SEQUENCE LISTING

<110> GENOPLANTE-VALOR
   FAVERY, Bruno
   ABAD, Pierre
   CAILLAUD, Marie-Cécile
<120> Method for increasing the resistance of a plant to endoparasitic nematodes
<130> MJP/XRN/mad-F1516/32-WO
<150> EP 07 290 610.0 <151> 2007-05-15
<160> 15
<170> PatentIn version 3.3
<210> 1
   <211> 2274
   <212> DNA
   <213> Arabidopsis thaliana
<220>
   <221> CDS
   <222> (1)..(2124)
<400> 1
   atg gca agt gtt caa aaa gat ccg att ctt caa gta gag aca act tgt       48
   Met Ala Ser Val Gln Lys Asp Pro Ile Leu Gln Val Glu Thr Thr Cys
   1               5                   10                  15
   gga tct ctt tta ttc gaa ctc cag att att tgg gat gaa gtt gga gaa       96
   Gly Ser Leu Leu Phe Glu Leu Gln Ile Ile Trp Asp Glu Val Gly Glu
               20                  25                  30
   aca gaa act gat aga gat caa atg ttg ctt gag ctt gaa cgt gaa tgt      144
   Thr Glu Thr Asp Arg Asp Gln Met Leu Leu Glu Leu Glu Arg Glu Cys
           35                  40                  45
   ttg gaa gtt tat aga aga aaa gtt gat caa gct aat cga tgt aga gct      192
   Leu Glu Val Tyr Arg Arg Lys Val Asp Gln Ala Asn Arg Cys Arg Ala
       50                  55                  60
   cag ctt aga caa gca ata gct gat gct gaa gca caa tta gct gca att      240
   Gln Leu Arg Gln Ala Ile Ala Asp Ala Glu Ala Gln Leu Ala Ala Ile
   65                  70                  75                  80
   tgt tct gct atg gga gag cgt cct gtg cat att aga cag tct gat caa      288
   Cys Ser Ala Met Gly Glu Arg Pro Val His Ile Arg Gln Ser Asp Gln
                   85                  90                  95
   agc gtt ggg agc ttg aag caa gag ctt ggg agg att ctt cct gaa ctg      336
   Ser Val Gly Ser Leu Lys Gln Glu Leu Gly Arg Ile Leu Pro Glu Leu
               100                 105                 110
   gaa gag atg caa aag agg aaa gtt gaa aga aga aac cag ttc att gtt      384
   Glu Glu Met Gln Lys Arg Lys Val Glu Arg Arg Asn Gln Phe Ile Val
           115                 120                 125
   gtc atg gag caa ata gat agt att act aat gat att aaa ggt caa gga      432
   Val Met Glu Gln Ile Asp Ser Ile Thr Asn Asp Ile Lys Gly Gln Gly
       130                 135                 140
   gaa ctt gtt cat tcc gaa cct ctt atc gat gag act aac ttg tct atg      480
   Glu Leu Val His Ser Glu Pro Leu Ile Asp Glu Thr Asn Leu Ser Met
   145                 150                 155                 160
   aga aag ctt gaa gag tta cac tgc cag ctg caa gta ctt cag aag gag      528
   Arg Lys Leu Glu Glu Leu His Cys Gln Leu Gln Val Leu Gln Lys Glu
                   165                 170                 175
   aag att gat cgt gtg gag acg att cgg aag cac ctg tgt act ctg tat      576
   Lys Ile Asp Arg Val Glu Thr Ile Arg Lys His Leu Cys Thr Leu Tyr
               180                 185                 190
   tcc cat tgt tca gta ctt gga atg gat ttt aat gag gtt gtt ggc caa      624
   Ser His Cys Ser Val Leu Gly Met Asp Phe Asn Glu Val Val Gly Gln
           195                 200                 205
   gtg aat ccc aca ttg agt gat cca gaa gga cca aga agt tta agt gat      672
   Val Asn Pro Thr Leu Ser Asp Pro Glu Gly Pro Arg Ser Leu Ser Asp
       210                 215                 220
   cat aca att gag aaa ttg ggg gct gca gtt cag aag ctg atg gag gtc      720
   His Thr Ile Glu Lys Leu Gly Ala Ala Val Gln Lys Leu Met Glu Val
   225                 230                 235                 240
   aag ata cag aga atg cag agg ctt caa gat cta gca aca acc atg ttg      768
   Lys Ile Gln Arg Met Gln Arg Leu Gln Asp Leu Ala Thr Thr Met Leu
                   245                 250                 255
   gag ctc tgg aat ttg atg gat aca ccc ata gaa gag cag caa gag tat      816
   Glu Leu Trp Asn Leu Met Asp Thr Pro Ile Glu Glu Gln Gln Glu Tyr
               260                 265                 270
   cag cat att acc tgc aat ata gct gca tca gaa cat gaa ata act gaa      864
   Gln His Ile Thr Cys Asn Ile Ala Ala Ser Glu His Glu Ile Thr Glu
           275                 280                 285
   gcc aat agt ctc tct gaa gac ttc att aaa tac gtt gag gca gaa gtt      912
   Ala Asn Ser Leu Ser Glu Asp Phe Ile Lys Tyr Val Glu Ala Glu Val
       290                 295                 300
   gtc cga ttg gac gag gta aaa gca agc aaa atg aaa gaa ctt gtg ttg      960
   Val Arg Leu Asp Glu Val Lys Ala Ser Lys Met Lys Glu Leu Val Leu
   305                 310                 315                 320
   aag aaa agg tca gaa cta gag gag ata tgt agg aaa acc cac ttg ttg     1008
   Lys Lys Arg Ser Glu Leu Glu Glu Ile Cys Arg Lys Thr His Leu Leu
                   325                 330                 335
   cca gtg tca gac agt gct ata gat cag act atc gtg gcg ata gaa tct     1056
   Pro Val Ser Asp Ser Ala Ile Asp Gln Thr Ile Val Ala Ile Glu Ser
               340                 345                 350
   ggt att gtg gat gct aca atg gtc ctg gag cat ctt gag caa cat ata     1104
   Gly Ile Val Asp Ala Thr Met Val Leu Glu His Leu Glu Gln His Ile
           355                 360                 365
   tct aag atc aaa gag gaa gca cta agc cga aaa gag ata ctt gaa agg      1152
   Ser Lys Ile Lys Glu Glu Ala Leu Ser Arg Lys Glu Ile Leu Glu Arg
       370                 375                 380
   gtt gag aaa tgg tta tct gcc tgc gat gaa gaa agt tgg ctt gaa gaa      1200
   Val Glu Lys Trp Leu Ser Ala Cys Asp Glu Glu Ser Trp Leu Glu Glu
   385                 390                 395                 400
   tac aac agg gat gat aac cga tac aat gct ggg cgt gga gct cat ctt      1248
   Tyr Asn Arg Asp Asp Asn Arg Tyr Asn Ala Gly Arg Gly Ala His Leu
                   405                 410                 415
   act ctc aag cgc gct gaa aaa gct cgt aat ctt gtt acc aag ctc cca      1296
   Thr Leu Lys Arg Ala Glu Lys Ala Arg Asn Leu Val Thr Lys Leu Pro
               420                 425                 430
   ggt atg gtt gaa gca ctg gcc tcg aag aca ata gtt tgg gaa caa gaa      1344
   Gly Met Val Glu Ala Leu Ala Ser Lys Thr Ile Val Trp Glu Gln Glu
           435                 440                 445
   aac ggg att gaa ttt ctc tat gat ggt atc cgt ctc ctt tct atg ctt      1392
   Asn Gly Ile Glu Phe Leu Tyr Asp Gly Ile Arg Leu Leu Ser Met Leu
       450                 455                 460
   gag gag tat aac ata ctc agg cag gaa aga gaa gaa gaa cac cgt cgg      1440
   Glu Glu Tyr Asn Ile Leu Arg Gln Glu Arg Glu Glu Glu His Arg Arg
   465                 470                 475                 480
   caa cgg gat cag aaa aag ctt caa gga cag ctc ata gca gaa caa gag      1488
   Gln Arg Asp Gln Lys Lys Leu Gln Gly Gln Leu Ile Ala Glu Gln Glu
                   485                 490                 495
   gca ctt tac ggg tcc aaa ccc agc cca tct aaa ccc ctt ggt ggt aaa      1536
   Ala Leu Tyr Gly Ser Lys Pro Ser Pro Ser Lys Pro Leu Gly Gly Lys
               500                 505                 510
   aag gct cca aga atg tcg acc ggt ggt gca tct aac aga aga ctt tcc      1584
   Lys Ala Pro Arg Met Ser Thr Gly Gly Ala Ser Asn Arg Arg Leu Ser
           515                 520                 525
   tta gga gca gca atg cat cag act cct aag cct aac aag aaa gca gat      1632
   Leu Gly Ala Ala Met His Gln Thr Pro Lys Pro Asn Lys Lys Ala Asp
       530                 535                 540
   cac cga cat aat gat gga gct tta tca aat gga aga aga ggg ctt gac      1680
   His Arg His Asn Asp Gly Ala Leu Ser Asn Gly Arg Arg Gly Leu Asp
   545                 550                 555                 560
   att gca gga cta ccc tca agg aaa caa tca atg aat cct tct gaa atg      1728
   Ile Ala Gly Leu Pro Ser Arg Lys Gln Ser Met Asn Pro Ser Glu Met
                   565                 570                 575
   ttg caa tcc cca tta gtt cgc aaa ccg ttt tcc ccc ata tcg acc acg      1776
   Leu Gln Ser Pro Leu Val Arg Lys Pro Phe Ser Pro Ile Ser Thr Thr
               580                 585                 590
   gtg gtt gca tca aag gcc aac ata gca aca aca aca aca caa caa ctt      1824
   Val Val Ala Ser Lys Ala Asn Ile Ala Thr Thr Thr Thr Gln Gln Leu
           595                 600                 605
   ccg aaa aac aac gca gtg aat gag atc tca tca ttt gca act cct atc      1872
   Pro Lys Asn Asn Ala Val Asn Glu Ile Ser Ser Phe Ala Thr Pro Ile
       610                 615                 620
   aag aac aac aac att ctc agg aac ttg gaa gaa gag aag atg atg act      1920
   Lys Asn Asn Asn Ile Leu Arg Asn Leu Glu Glu Glu Lys Met Met Thr
   625                 630                 635                 640
   atg atg atg caa aca ccg aaa aat gtt gct gca atg att cca atc ccg      1968
   Met Met Met Gln Thr Pro Lys Asn Val Ala Ala Met Ile Pro Ile Pro
                   645                 650                 655
   tcg aca cca gca aca gtc tct gtc cca atg cac acc gca cca aca cca      2016
   Ser Thr Pro Ala Thr Val Ser Val Pro Met His Thr Ala Pro Thr Pro
               660                 665                 670
   ttc acc aac aat gca aga ttg atg tct gaa aag cct gaa gtt gtt gag      2064
   Phe Thr Asn Asn Ala Arg Leu Met Ser Glu Lys Pro Glu Val Val Glu
           675                 680                 685
   tac tcg ttt gag gaa cga aga ctc gcc ttc atg cta cag tct gaa tgt      2112
   Tyr Ser Phe Glu Glu Arg Arg Leu Ala Phe Met Leu Gln Ser Glu Cys
       690                 695                 700
   cgt ttg gtt tga tcatcaatcc gttggattca acttaagttc ttttatttgt          2164
   Arg Leu Val
   705
   aaaactcaaa ttgaatgtgg tttggtttgt aaaaggtcaa ttatatgatt ggcttttttt   2224
   tttttttttg agtaatcatt tttacacgta tggtttattc tgagactgac                2274
<210> 2
   <211> 707
   <212> PRT
   <213> Arabidopsis thaliana
<400> 2
   Met Ala Ser Val Gln Lys Asp Pro Ile Leu Gln Val Glu Thr Thr Cys
   1               5                   10                  15
   Gly Ser Leu Leu Phe Glu Leu Gln Ile Ile Trp Asp Glu Val Gly Glu
               20                  25                  30
   Thr Glu Thr Asp Arg Asp Gln Met Leu Leu Glu Leu Glu Arg Glu Cys
           35                  40                  45
   Leu Glu Val Tyr Arg Arg Lys Val Asp Gln Ala Asn Arg Cys Arg Ala
       50                  55                  60
   Gln Leu Arg Gln Ala Ile Ala Asp Ala Glu Ala Gln Leu Ala Ala Ile
   65                  70                  75                  80
   Cys Ser Ala Met Gly Glu Arg Pro Val His Ile Arg Gln Ser Asp Gln
                   85                  90                  95
   Ser Val Gly Ser Leu Lys Gln Glu Leu Gly Arg Ile Leu Pro Glu Leu
               100                 105                 110
   Glu Glu Met Gln Lys Arg Lys Val Glu Arg Arg Asn Gln Phe Ile Val
           115                 120                 125
   Val Met Glu Gln Ile Asp Ser Ile Thr Asn Asp Ile Lys Gly Gln Gly
       130                 135                 140
   Glu Leu Val His Ser Glu Pro Leu Ile Asp Glu Thr Asn Leu Ser Met
   145                 150                 155                 160
   Arg Lys Leu Glu Glu Leu His Cys Gln Leu Gln Val Leu Gln Lys Glu
                   165                 170                 175
   Lys Ile Asp Arg Val Glu Thr Ile Arg Lys His Leu Cys Thr Leu Tyr
               180                 185                 190
   Ser His Cys Ser Val Leu Gly Met Asp Phe Asn Glu Val Val Gly Gln
           195                 200                 205
   Val Asn Pro Thr Leu Ser Asp Pro Glu Gly Pro Arg Ser Leu Ser Asp
       210                 215                 220
   His Thr Ile Glu Lys Leu Gly Ala Ala Val Gln Lys Leu Met Glu Val
   225                 230                 235                 240
   Lys Ile Gln Arg Met Gln Arg Leu Gln Asp Leu Ala Thr Thr Met Leu
                   245                 250                 255
   Glu Leu Trp Asn Leu Met Asp Thr Pro Ile Glu Glu Gln Gln Glu Tyr
               260                 265                 270
   Gln His Ile Thr Cys Asn Ile Ala Ala Ser Glu His Glu Ile Thr Glu
           275                 280                 285
   Ala Asn Ser Leu Ser Glu Asp Phe Ile Lys Tyr Val Glu Ala Glu Val
       290                 295                 300
   Val Arg Leu Asp Glu Val Lys Ala Ser Lys Met Lys Glu Leu Val Leu
   305                 310                 315                 320
   Lys Lys Arg Ser Glu Leu Glu Glu Ile Cys Arg Lys Thr His Leu Leu
                   325                 330                 335
   Pro Val Ser Asp Ser Ala Ile Asp Gln Thr Ile Val Ala Ile Glu Ser
               340                 345                 350
   Gly Ile Val Asp Ala Thr Met Val Leu Glu His Leu Glu Gln His Ile
           355                 360                 365
   Ser Lys Ile Lys Glu Glu Ala Leu Ser Arg Lys Glu Ile Leu Glu Arg
       370                 375                 380
   Val Glu Lys Trp Leu Ser Ala Cys Asp Glu Glu Ser Trp Leu Glu Glu
   385                 390                 395                 400
   Tyr Asn Arg Asp Asp Asn Arg Tyr Asn Ala Gly Arg Gly Ala His Leu
                   405                 410                 415
   Thr Leu Lys Arg Ala Glu Lys Ala Arg Asn Leu Val Thr Lys Leu Pro
               420                 425                 430
   Gly Met Val Glu Ala Leu Ala Ser Lys Thr Ile Val Trp Glu Gln Glu
           435                 440                 445
   Asn Gly Ile Glu Phe Leu Tyr Asp Gly Ile Arg Leu Leu Ser Met Leu
       450                 455                 460
   Glu Glu Tyr Asn Ile Leu Arg Gln Glu Arg Glu Glu Glu His Arg Arg
   465                 470                 475                 480
   Gln Arg Asp Gln Lys Lys Leu Gln Gly Gln Leu Ile Ala Glu Gln Glu
                   485                 490                 495
   Ala Leu Tyr Gly Ser Lys Pro Ser Pro Ser Lys Pro Leu Gly Gly Lys
               500                 505                 510
   Lys Ala Pro Arg Met Ser Thr Gly Gly Ala Ser Asn Arg Arg Leu Ser
           515                 520                 525
   Leu Gly Ala Ala Met His Gln Thr Pro Lys Pro Asn Lys Lys Ala Asp
       530                 535                 540
   His Arg His Asn Asp Gly Ala Leu Ser Asn Gly Arg Arg Gly Leu Asp
   545                 550                 555                 560
   Ile Ala Gly Leu Pro Ser Arg Lys Gln Ser Met Asn Pro Ser Glu Met
                   565                 570                 575
   Leu Gln Ser Pro Leu Val Arg Lys Pro Phe Ser Pro Ile Ser Thr Thr
               580                 585                 590
   Val Val Ala Ser Lys Ala Asn Ile Ala Thr Thr Thr Thr Gln Gln Leu
           595                 600                 605
   Pro Lys Asn Asn Ala Val Asn Glu Ile Ser Ser Phe Ala Thr Pro Ile
       610                 615                 620
   Lys Asn Asn Asn Ile Leu Arg Asn Leu Glu Glu Glu Lys Met Met Thr
   625                 630                 635                 640
   Met Met Met Gln Thr Pro Lys Asn Val Ala Ala Met Ile Pro Ile Pro
                   645                 650                 655
   Ser Thr Pro Ala Thr Val Ser Val Pro Met His Thr Ala Pro Thr Pro
               660                 665                 670
   Phe Thr Asn Asn Ala Arg Leu Met Ser Glu Lys Pro Glu Val Val Glu
           675                 680                 685
   Tyr Ser Phe Glu Glu Arg Arg Leu Ala Phe Met Leu Gln Ser Glu Cys
       690                 695                 700
   Arg Leu Val
   705
<210> 3
   <211> 20
   <212> DNA
   <213> Artificial
<220>
   <223> primer GUS
<400> 3
   tccagactga atgcccacag 20
<210> 4
   <211> 22
   <212> DNA
   <213> Artificial
<220>
   <223> Primer DYCR
<400> 4
   gcagttcaga agctgatgga gg 22
<210> 5
   <211> 23
   <212> DNA
   <213> Artificial
<220>
   <223> Primer DYC5RC
<400> 5
   cctgcctgag tatgttatac tcc 23
<210> 6
   <211> 23
   <212> DNA
   <213> Artificial
<220>
   <223> Primer DYC6
<400> 6
   ggagtataac atactcaggc agg 23
<210> 7
   <211> 25
   <212> DNA
   <213> Artificial
<220>
   <223> Primer DYC9RC
<400> 7
   gatgatcaaa ccaaacgaca ttcag 25
<210> 8
   <211> 38
   <212> DNA
   <213> Artificial
<220>
   <223> Primer Gw5pdyc
<400> 8
   aaaaagcagg cttcacactc ttccctacac aaaaccgc 38
<210> 9
   <211> 40
   <212> DNA
   <213> Artificial
<220>
   <223> Primer Gw3pdyc
<400> 9
   agaaagctgg gtgttcgaaa tgcttaagcc tgtaacaggg 40
<210> 10
   <211> 41
   <212> DNA
   <213> Artificial
<220>
   <223> Primer Gw5dycB
<400> 10
   aaaaagcagg cttcaccatg gcaagtgttc aaaaagatcc g 41
<210> 11
   <211> 37
   <212> DNA
   <213> Artificial
<220>
   <223> Primer Gw3dycK
<400> 11
   agaaagctgg gtgtcaaacc aaacgacatt cagactg 37
<210> 12
   <211> 34
   <212> DNA
   <213> Artificial
<220>
   <223> Primer Gw3dycL
<400> 12
   agaaagctgg gtgaaccaaa cgacattcag actg 34
<210> 13
   <211> 449
   <212> DNA
   <213> Arabidopsis thaliana
<400> 13
   cccaaaacct tttacttctt cgtaacggct ctatttctct tctctcttct ctccacacac 60
   agaaatatct cgatctaggt ttctaaaatt ggattctcta tctcaaacgc taatcgaaga    120
   gttttctctg aatcgttttc ttaaatctaa tctcgcgaag tttcagtgac agattcaatc    180
   aaacttcaaa atcatcatca gttctggaaa atttacgaga aatctcccgt ttcaggcaag    240
   ttttctctca atttgttgct tcgcaatgtc aaaatcaatc atataggctt tcaatcaatt    300
   gcttcgaatt tagcttctaa ttttgagaat gacatagtta gggtttcgtt ttccctgtta    360
   caggcttaag catttcgaaa tggcaagtgt tcaaaaagat ccgattcttc aagtagagac    420
   aacttgtgga tctcttttat tcgaactcc                                      449
<210> 14
   <211> 36
   <212> DNA
   <213> Artificial
<220>
   <223> Primer MAP_RNAiGM5
<400> 14
   aaaaagcagg cttccccaaa accttttact tcttcg 36
<210> 15
   <211> 36
   <212> DNA
   <213> Artificial
<220>
   <223> Primer MAP_RNAiGW3
<400> 15
   agaaagctgg gtgggagttc gaataaaaga gatcca 36